# EUROPEAN PATENT SPECIFICATION

(11) **EP 2 788 001 B1**
(45) Date of publication and mention of the grant of the patent: **02.12.2015**
(21) Application number: 12797884.9
(22) Date of filing: 04.12.2012
(51) Int. Cl.: A61K 31/55, A61K 31/573, A61K 9/00, A61K 9/12, A61M 11/04, A61M 11/06, A61M 15/00, A61M 15/08, A61P 27/14

(54) **NASAL FORMULATION**
FORMULIERUNG ZUR NASALEN VERABREICHUNG
FORMULATION NASALE

(30) Priority: 07.12.2011 US 201161567893 P; 19.12.2011 GB 201121812
(43) Date of publication of application: 15.10.2014
(73) Proprietor: Teva Branded Pharmaceutical Products R & D, Inc., Frazer, Pennsylvania 19355 (US)
(72) Inventor: ZENG, Xian-Ming, Miami, Florida 33169-5043 (US); LY, Jade Ching-Ying, Miami, Florida 33169-5043 (US); DALVI, Mukul C, Miami, Florida 33169-5043 (US)
(74) Representative: Cottam, David William
(86) International application number: PCT/EP2012/074374
(87) International publication number: WO 2013/083569

(56) References cited:
- WO-A1-2008/023018
- WO-A1-2009/046074
- WO-A1-2012/048867
- WO-A2-2006/102494
- GB-A- 2 389 530

## Description

This invention relates to a nasal formulation and particularly to a nasal formulation containing azelastine and beclomethasone dipropionate.

Azelastine is a known histamine-H₁-receptor antagonist and is classified as a potent long-acting anti-allergy compound. It has the following chemical structure:

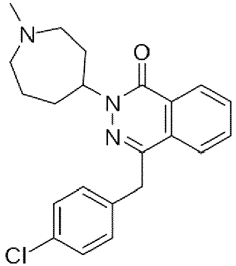

Azelastine is administered nasally and is indicated for the treatment of both seasonal allergic rhinitis (e.g. hay fever) and perennial allergic rhinitis. The commercial product on the market containing azelastine is Rhinolast® Nasal Spray. This formulation contains azelastine hydrochloride, citric acid and water. The formulation is administered from a pump dispenser.

Beclomethasone dipropionate (BDP) is a known glucocorticosteroid and has the following chemical structure:

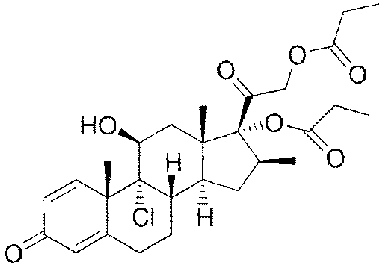

BDP may also be administered nasally and, when administered in this manner, is indicated for the prevention and treatment of allergic rhinitis, including hay fever. The commercial product on the market containing BDP is Beconase® Hayfever Nasal Spray. This formulation contains Avicel RC 591 (microcrystalline cellulose and carboxymethylcellulose sodium), anhydrous dextrose, benzalkonium chloride (added as benzalkonium chloride solution), phenylethyl alcohol, polysorbate 80 and purified water. The formulation is administered via a metering, atomising pump and nasal applicator.

WO 2005/027839 discloses the combination of an antihistamine, e.g. azelastine, and a steroid, e.g. BDP. It is beneficial to administer azelastine and BDP in combination in order to increase patient convenience and hence compliance with the treatment. However, WO 2005/027839 is not specifically directed to the combination of azelastine and BDP and provides no general guidance on formulations nor any consideration of formulating this specific combination. Moreover, routes of administration for the various embodiments include topical, transdermal, nasal and systemic administration (e.g. intravenous, intramuscular, subcutaneous, inhalation, rectal, buccal, vaginal, intraperitoneal, intraarticular, ophthalmic or oral administration) and there is no particular guidance on nasal formulations. GB 2389 530 discloses a preparation comprising azelastine hydrochloride and beclomethasone dipropionate.

WO 2012/048867 discloses a nasal spray device and WO 2008/023018 discloses an actuator for an inhaler.

There remains, therefore, a need for suitable formulations for administering this combination product.

Accordingly, the present invention provides a solution formulation for nasal administration comprising azelastine, beclomethasone dipropionate, a co-solvent, hydrochloric acid and an HFA propellant, wherein the molar ratio of azelastine free base to hydrochloric acid is 6:1 to 15:1.

The present invention will now be described with reference to the accompanying drawings, in which:
Fig. 1 shows a cut-away perspective schematic view of a conventional nasal spray device as used with the present invention; and
Fig. 2 shows the results of a 21 day stability study of a formulation according to the present invention.

Nasal spray devices include unit-dose (single use) devices having syringe-like mechanisms and metered-dose devices intended for multiple usage cycles. Unit dose devices are appropriate for delivering certain medicaments such as vaccines, whereas metered-dose devices are more suited to long-term dosage regimes, for example for the treatment of rhinitis. The commercially available metered-dose devices discussed hereinabove comprise a vial containing an aqueous suspension of the medicament. The vial is provided with a manually operated pump adapted to atomise metered doses of the medicament formulation for delivery to the nasal cavity. Further examples of this type of nasal spray device include Flixonase® (fluticasone propionate, GSK), Nasacort AQ® (triamcinolone acetoinide, Sanofi-Aventis) and Nasonex® (momethasone furoate monohydrate, Schering-Plough).

Although nasal spray devices having manually operated pumps have achieved some success in the marketplace, they have a number of drawbacks. For example, manually operated pumps have a relatively large actuation force which may, for some users, such as the very young and the elderly, be difficult to achieve on a repeatable basis. Variations in the applied actuation force can lead to some users receiving medicament doses with less than optimal spray characteristics. Moreover, aqueous-based nasal sprays tend to suffer from post-nasal drip which is a particular problem for azelastine as it has a strong and unpleasant taste.

To address the problems associated with these known metered-dose nasal spray devices, the present inventors have replaced the manually operated pump with a pressurised aerosol canister. A typical aerosol canister comprises a cylindrical vial containing the medicament. The medicament is typically an active ingredient together with a suitable propellant. The medicament may be in the form of a solution or a suspension in the propellant and excipients may be added to facilitate dissolution of the active ingredient (e.g. co-solvents) or to stabilise the suspension (e.g. surfactants). The vial is provided with a metering valve having an axially extending valve stem. Displacement of the valve stem relative to the vial causes the dispensation of a metered dose of the medicament formulation as an aerosol. Compared to manually operated pumps, pressurised aerosol canisters require low actuation forces, provide consistent aerosol characteristics, and do not suffer from the problem of post-nasal drip.

However, a significant problem in providing a combined formulation of azelastine and BDP is that BDP is an ester and hence is unstable in the presence of acid or base (esters are well known to hydrolyse to the free acid in the presence of acid or base) and azelastine is a basic molecule. Azelastine has been marketed as the hydrochloride salt, but the problem is that the hydrochloride salt is not soluble in an HFA/ethanol formulation.

One option taken in the area of inhalable medicaments has been to formulate the BDP as a suspension. Ventide® is a suspension formulation of BDP and salbutamol sulfate. The salbutamol has to be present as the sulfate salt because salbutamol is basic and would destabilise the BDP. To address this problem, the product contains an insoluble salbutamol salt rather than the soluble free base. That is, the formulator decided to use a suspension to solve the problem of chemical instability. However, suspension formulations are less physically stable than solutions and have a tendency to agglomerate and also to block the narrow orifice in the stem block of the device.

It has now been found that a combination product containing these active ingredients can be prepared by providing a precisely defined ratio of azelastine to acid such that the azelastine is both soluble in HFA/ethanol and does not chemically degrade the BDP, allowing the product to be formulated as a solution. This allows the combination of azelastine and BDP advantageously to be formulated as an HFA solution formulation.

A therapeutically effective amount of the active ingredient needs to be delivered. The azelastine is preferably present in an amount to provide a dosage per actuation of 0.1-1 mg, more preferably 0.3-0.8 mg. The formulation will typically contain 0.20-1.00% of azelastine, calculated as the free base and based on the total weight of the formulation. The BDP is preferably present in an amount to provide a dosage per actuation of 10-200 g, more preferably 30-100 g. The formulation will typically contain 0.02 -0.60% of BDP based on the total weight of the formulation.

The formulation contains azelastine which is present in the form of both azelastine free base and an azelastine salt. This form of azelastine is prepared by combining azelastine free base with an acid. The acid is hydrochloric acid, and hence the salt is the hydrochloride salt. The molar ratio of azelastine free base to hydrochloric acid is 6:1 to 15:1, preferably 9:1 to 12:1. This provides further improvements to stability. In an alternative, but less preferred, embodiment the molar ratio of azelastine free base to hydrochloric acid is 9:1 to 10:9, or 2.5:1 to 4:1. A molar ratio is quoted rather than a pH value because an HFA solution provides a non-aqueous environment which renders a measure of pH inappropriate.

Typically, concentrated hydrochloric acid is used, for example 12 N hydrochloric acid. An example of a suitable formulation using 12 N hydrochloric acid is as follows:

| **Component** | **Amount (% w/w)** | **Amount per can (mg)** |
|---|---|---|
| Azelastine, free base | 0.406 | 41.45 |
| BDP | 0.169 | 17.20 |
| HCl, 12 N | 0.008-0.039 | 0.79-3.95 |
| Dehydrated ethanol | 7.998 | 815.56 |
| HFA 134a | qs | qs |
| Total | 100 | 10,200 |

The amounts of hydrochloric acid added to the formulation range form 0.007-0.018% w/w, which corresponds to a ratio of 15:1 to 6:1. The amount of acid may be varied within this range to provide other ratios. The effect is that the azelastine is present in the form of both azelastine free base and an azelastine salt, with hydrochloric acid, the hydrochloride salt.

The water present in the formulation may accelerate the degradation and hence it is preferred to have low levels of water. Preferably water is present at 0.01-0.20 wt%, more preferably 0.02-0.05 wt%, based on the total weight of the formulation.

The propellant of the pharmaceutical solution of the present invention is a hydrofluoroalkane (HFA) propellant, more preferably an HFA propellant selected from P134a (1,1,1,2-tetrafluoroethane), P227 (1,1,1,2,3,3,3-heptafluoropropane) or mixtures thereof. Other hydrofluorocarbons, hydrocarbons or aliphatic gases (e.g. butane or dimethylether) may be added to modify the propellant characteristics as required. However, it is preferred that P134a and/or P227 are the sole propellants present and most preferably P134a is the sole propellant present. The propellant preferably constitutes 80% to 99% w/w, more preferably 90 to 98% w/w, based on the total weight of the solution.

The co-solvent is present in order to solubilise the active ingredients. The co-solvent is preferably a C₂₋₆ aliphatic alcohol, such as ethanol or isopropyl alcohol, and preferably ethanol. The co-solvent is present in an amount sufficient to dissolve the medicaments present in the formulation and to maintain the medicaments dissolved over the time period and conditions experienced by commercial aerosol products. Preferably the solvent is present in an amount to prevent precipitation of the active ingredient even at temperatures down to -20 °C. The co-solvent is typically present at 1-20% w/w, more preferably 6-15% w/w and most preferably 7-10% w/w, based on the total weight of the solution. A particularly preferred embodiment uses ethanol in a range of 1-20% w/w, more preferably 6-15% w/w and most preferably 7-10% w/w, based on the total weight of the solution.

In a specific embodiment of the present invention, the pharmaceutical solution comprises azelastine, beclomethasone dipropionate, ethanol, hydrochloric acid and a propellant selected from 1,1,1,2-tetrafluoroethane (P134a), 1,1,1,2,3,3,3-heptafluoropropane (P227) and a mixture thereof.

In a preferred embodiment, the formulation of the present invention comprises azelastine, beclomethasone dipropionate, ethanol, hydrochloric acid and a propellant selected from 1,1,1,2-tetrafluoroethane (P134a), 1,1,1,2,3,3,3-heptafluoropropane (P227) and a mixture thereof, wherein the molar ratio of azelastine free base to hydrochloric acid is 6:1 to 15:1.

A preferred solution formulation according to the present invention comprises 0.20-1.00% of azelastine, calculated as the free base, 0.02-0.6% w/w beclomethasone dipropionate, 1% to 20% w/w ethanol, hydrochloric acid and 80 to 99% w/w of propellant, wherein the percentages by weight are based on the total weight of the solution aerosol and the molar ratio of azelastine free base to hydrochloric acid is 6:1 to 15:1. A particularly preferred solution consists essentially of these components and more preferably the solution consists of these components.

The pharmaceutical solution of the present invention is preferably substantially free of surfactant. Surfactants are often added to suspensions to stabilise the suspension. However, since the formulation of the present invention is a solution, a surfactant is not required. Nevertheless, small quantities can be tolerated without adversely affecting the formulation. Preferably the formulation contains no more than 0.0005% w/w of a surfactant based on the total weight of the solution. Preferred formulations contain no surfactant. The presence of a significant amount of a surfactant is believed to be undesirable for solution formulations of beclomethasone dipropionate because surfactants such as oleic acid and lecithin are believed to promote chemical degradation of the active ingredient when the latter is dissolved in the mixture of the propellant and ethanol.

The formulation of the present invention is intended to be used for the prophylaxis and/or treatment of seasonal allergic rhinitis (including hay fever) and perennial rhinitis.

The pharmaceutical solution of the present invention may be prepared by dissolving the desired amount of active ingredients/acid in the desired amount of co-solvent accompanied by stirring or sonication. The aerosol canister may then be filled using conventional cold-fill or pressure-fill methods.

The present invention also provides a nasal spray device for the delivery of a pharmaceutical solution to the nasal cavity in metered doses, wherein the pharmaceutical solution is the formulation described herein. Preferably, the device comprises a pressurised aerosol canister including a vial containing the pharmaceutical solution, the aerosol canister further including a metering valve having a valve stem; and an actuator for the aerosol canister, the actuator including a stem block having a receptacle into which the valve stem of metering valve of the aerosol canister is received and axially located and being displaceable relative to the vial of the aerosol canister to actuate the metering valve of the aerosol canister, a sump extending below the receptacle, the stem block further defining a discharge orifice for the pharmaceutical solution and a transfer channel through which a dispensed dose of the pharmaceutical solution is able to pass from the sump to the discharge orifice, wherein the actuator further comprises a delivery outlet for the aerosol plume, the discharge orifice being arranged to direct the aerosol plume through the delivery outlet.

The device comprises a pressurised aerosol canister. Such canisters are known in the art and are commercially available. The aerosol canister is typically composed of aluminium or an aluminium alloy. The internal surfaces of the aerosol canister may be coated with a fluorocarbon polymer, such as PTFE or FEP, optionally together with non-fluorinated polymer to promote adhesion, such as PES. The canister includes a vial containing a pharmaceutical solution comprising an active ingredient and a propellant. The aerosol canister further includes a metering valve having a valve stem axially displaceable relative to the vial to cause the dispensation of a metered dose of the pharmaceutical solution through the valve stem. The device also comprises an actuator for the aerosol canister including a stem block having a receptacle into which the valve stem of the aerosol canister is received and axially located, and being displaceable relative to the vial of the aerosol canister to actuate the metering valve of the aerosol canister. The stem block further defines a discharge nozzle for the pharmaceutical solution and a channel through which a dispensed dose of the pharmaceutical solution is able to pass from the valve stem to the discharge orifice. The actuator further comprises a delivery outlet, such as a nose piece, for the aerosol plume, the discharge orifice being arranged to direct the aerosol plume through the delivery outlet.

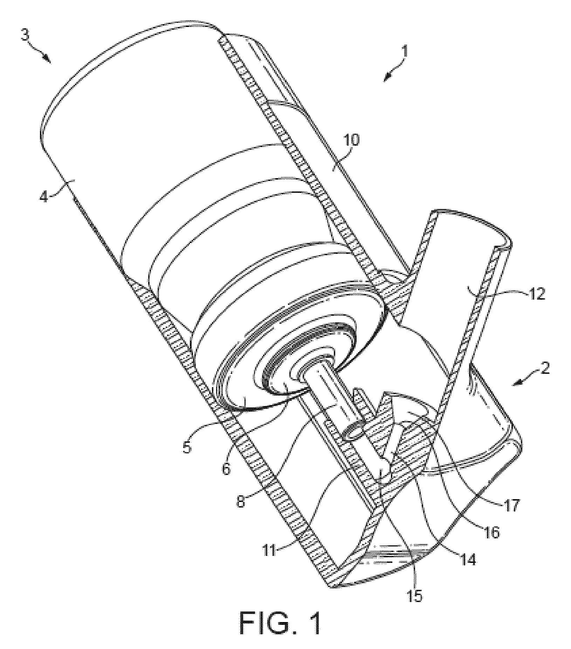

With reference to Fig. 1, a nasal spray device 1 according to the present invention is based on a conventional pressurised metered dose inhaler (pMDI), but modified for nasal use rather than for inhalation via the mouth. Accordingly, the device 1 comprises an actuator 2 accommodating an aerosol canister 3 containing a pharmaceutical solution for delivery to the nasal cavity of a user.

The aerosol canister 3 is constructed to a standard design and specification and comprises a substantially cylindrical vial body 4 which contains the pharmaceutical solution. The aerosol canister 3 is charged with a pharmaceutical solution as described hereinabove. The vial body 4 is provided with a ferrule 5 which is crimped over a lip of the body to seal hermetically the pharmaceutical solution under pressure.

The ferrule 5 of the aerosol canister 3 is provided with a metering valve 6 designed to deliver a metered amount of the pharmaceutical solution to the user for each actuation of the valve 6. The metering valve 6 is of a known type available from manufacturers such as Consort Medical plc and 3M Drug Delivery Systems. See WO 99/47195 for further details of the metering valve suitable for use in the device of the present invention. The valve 6 generally comprises a metering chamber (not visible in Fig. 1, but shown in WO 99/47195) and a valve stem 8 in the form of a narrow tube protruding outwardly from ferrule 5. The metering valve 6 is actuated by displacing the valve stem 8 into the valve body against the action of a valve spring to allow the metered amount of the pharmaceutical solution to vent from the metering chamber through the stem 8. The propellant component of the pharmaceutical solution causes atomisation of the active ingredient by vaporising on release to the atmosphere. The metering chamber is then recharged with the pharmaceutical solution as the valve stem 8 is allowed to return to its starting position under the action of the valve spring.

With further reference to Fig. 1, the aerosol canister 3 is received into the open end of a body 10 of the actuator 2, with the valve stem 8 being received into and axially located by a stem block 11 of the actuator 2. The actuator body 10 is a moulded plastics component and the stem block 11 is formed as a protrusion which stands from the closed end of the actuator body 10. The stem block 11 includes a cylindrical receptacle configured for an interference fit with the valve stem 8 of the aerosol canister 3. The actuator body 10 generally defines a sleeve-like portion having a substantially circular cross-section, within which sleeve-like portion the aerosol canister 3 is axially displaceable relative to the stem block 11 and valve stem 8 to actuate the metering valve 6. A portion of the aerosol canister 3 at its non-valve end remains exposed in use so that the user is able to apply a manual pressure to displace the aerosol canister relative to the valve stem.

Although similar in the above-described respects, the nasal spray device 1 according to the present invention differs from conventional pMDIs in that the actuator body 10 defines a delivery outlet in the form of a nose piece 12 (rather than a mouth piece) for delivering the atomised pharmaceutical solution to the nasal cavity. The delivery outlet may be a tubular nose piece adapted for insertion into the nostril, and a circular end of the nose piece may have an inner diameter of 5 to 7.5 mm, preferably about 7.2 mm. The delivery outlet, the delivery orifice and the transfer channel may be aligned with each other, that is to say they may have substantially identical axes. The axis of the delivery outlet may be substantially perpendicular, or at an angle of up to 20º to the perpendicular, to the aerosol canister and the receptacle of the stem block. Preferably an axis of the nose piece 12 defines an angle of about 80º with the sleeve-like portion of the actuator body 10. The nose piece 12 directly faces the stem block 11 so that an aerosol plume produced at the valve stem can be delivered through the nose piece 12 into the nasal cavity.

The stem block 11 is moulded with a discharge orifice 17 facing the delivery outlet, and the discharge orifice 17 is fluidly connected to the receptacle of the stem block 11 so that the pharmaceutical solution is able to pass from the aerosol canister out through the nose piece 12, and into the nasal cavity of a user. In Fig. 1, the stem block 11 is shown having a channel 14 extending from a sump 15 underneath the receptacle of the stem block 11 into an opening 16. The sump 15 is preferably rounded to help to prevent blockages.

### Examples

### Example 1

A formulation in accordance with the present invention was prepared as follows:

| **Component** | **Amount (% w/w)** | **Amount per can (mg)** |
|---|---|---|
| Azelastine, free base | 0.406 | 41.45 |
| BDP | 0.169 | 17.20 |
| HCl, 12 N | 0.008 | 0.79 |
| Dehydrated ethanol | 7.998 | 815.56 |
| HFA 134a | qs | qs |
| Total | 100 | 10,200 |

The molar ratio of azelastine to hydrochloric acid in this formulation was 14:1.

### Example 2

A preferred formulation has a molar ratio of azelastine to hydrochloric acid of 9:1:

| **Component** | **Amount (% w/w)** | **Amount per can (mg)** |
|---|---|---|
| Azelastine, free base | 0.406 | 41.45 |
| BDP | 0.169 | 17.20 |
| HCl, 12 N | 0.012 | 1.20 |
| Dehydrated ethanol | 8.000 | 816.00 |
| HFA 134a | qs | qs |
| Total | 100 | 10,200 |

### Example 3

Further formulations were prepared containing the active ingredient(s) and ethanol with and without hydrochloric acid in order to test the stability of the active ingredients. The formulations were:
Formulation (i): azelastine + BDP, with acid
Formulation (ii): BDP without acid
Formulation (iii): BDP with acid
Formulation (iv): azelastine + BDP, without acid

The precise ethanol formulations were as follows:

| **Component (mg)** | **Amount (mg)** | | | |
|---|---|---|---|---|
| | **Formulation (i)** | **Formulation (ii)** | **Formulation (iii)** | **Formulation (iv)** |
| Azelastine, free base | 25.40 | - | - | 25.40 |
| BDP | 13.53 | 13.53 | 13.53 | 13.53 |
| HCl, 12 N | 0.40 | - | 0.40 | - |
| Dehydrated ethanol | 835.63 | qs | qs | qs |
| Total | 875.00 | 875.00 | 875.00 | 875.00 |

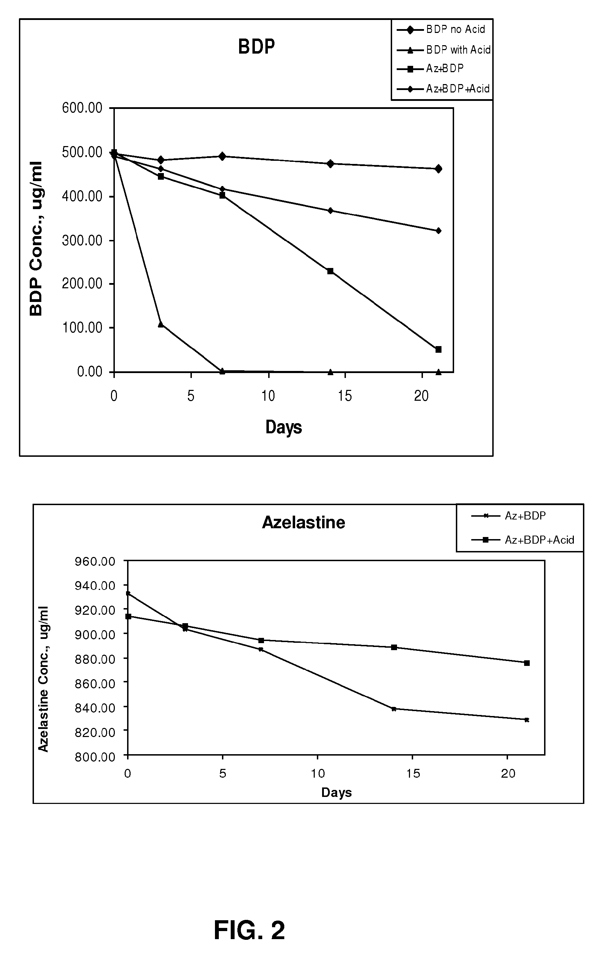

The formulations were stored at 60ºC for 21 days and the amounts of the active ingredients measured at intervals during the test period. The amounts of the active ingredients were measured using HPLC with external standards of azelastine free base and BDP. The results are shown graphically in Fig. 2. The results for BDP show that the formulations containing BDP and either hydrochloric acid or azelastine were chemically unstable (on account of ester hydrolysis caused by the acid/base). The BDP with no acid and no azelastine was stable. However, the combination of BDP with both acid and azelastine is stable. It is also noteworthy that some additional stabilisation of the azelastine was detected.

### Example 4

Further formulations were prepared containing the active ingredient(s) and ethanol as follows:

| **Az:HCl molar ratio** | **6:1** | | **9:1** | | **12:1** | | **15:1** | |
|---|---|---|---|---|---|---|---|---|
| | **Amount / can (mg)** | **% w/w** | **Amount / can (mg)** | **% w/w** | **Amount / can (mg)** | **% w/w** | **Amount / can** (**mg**) | **% w/w** |
| Azelastine, free base | 41.45 | 0.406 | 41.45 | 0.406 | 41.45 | 0.406 | 41.45 | 0.406 |
| BDP | 17.20 | 0.169 | 17.20 | 0.169 | 17.20 | 0.169 | 17.20 | 0.169 |
| HCl, 12 N | 1.81 | 0.018 | 1.20 | 0.012 | 0.90 | 0.009 | 0.72 | 0.007 |
| Ethanol | 816.00 | 8.000 | 816.00 | 8.000 | 816.00 | 8.000 | 816.00 | 8.000 |
| HFA 134a | 9323.54 | 91.41 | 9324.15 | 91.41 | 9324.45 | 91.42 | 9324.63 | 91.42 |
| Total (Nominal) | 10200.00 | 100.00 | 10200.00 | 100.00 | 10200.00 | 100.00 | 10200.00 | 100.00 |

The formulations were stored at 60ºC for two weeks. The amounts of the active ingredients and impurity levels were determined at 4, 7 and 14 days. All formulations showed favourable stability, although the best results were obtained with the 12:1 formulation.

### Example 5

Further formulations were prepared containing the active ingredient(s) and ethanol, with high and low water contents. The formulations were as follows:

| **Az:HCl molar ratio** | **6:1** | | **9:1** | | **12:1** | | **15:1** | |
|---|---|---|---|---|---|---|---|---|
| | **Amount / can (mg)** | **% w/w** | **Amount / can (mg)** | **% w/w** | **Amount / can (mg)** | **% w/w** | **Amount / can (mg)** | **% w/w** |
| Azelastine, free base | 41.45 | 0.406 | 41.45 | 0.406 | 41.45 | 0.406 | 41.45 | 0.406 |
| BDP | 17.20 | 0.169 | 17.20 | 0.169 | 17.20 | 0.169 | 17.20 | 0.169 |
| HCl, 12N | 1.81 | 0.018 | 1.20 | 0.012 | 0.90 | 0.009 | 0.72 | 0.007 |
| Water | 3.23 | 0.032 | 3.61 | 0.035 | 3.80 | 0.037 | 3.92 | 0.038 |
| Ethanol | 816.00 | 8.000 | 816.00 | 8.000 | 816.00 | 8.000 | 816.00 | 8.000 |
| HFA 134a | 9320.32 | 91.38 | 9320.54 | 91.38 | 9320.65 | 91.38 | 9320.71 | 91.38 |
| Total (Nominal) | 10200.00 | 100.00 | 10200.00 | 100.00 | 10200.00 | 100.00 | 10200.00 | 100.00 |

| **Az:HCl molar ratio** | **6:1** | | **9:1** | | **12:1** | | **15:1** | |
|---|---|---|---|---|---|---|---|---|
| | **Amount / can (mg)** | **% w/w** | **Amount / can (mg)** | **% w/w** | **Amount / can (mg)** | **% w/w** | **Amount / can (mg)** | **% w/w** |
| Azelastine, free base | 41.45 | 0.406 | 41.45 | 0.406 | 41.45 | 0.406 | 41.45 | 0.406 |
| BDP | 17.20 | 0.169 | 17.20 | 0.169 | 17.20 | 0.169 | 17.20 | 0.169 |
| HCl, 12 N | 1.81 | 0.018 | 1.20 | 0.012 | 0.90 | 0.009 | 0.72 | 0.007 |
| Water | 16.35 | 0.160 | 16.74 | 0.164 | 16.93 | 0.166 | 17.04 | 0.167 |
| Ethanol | 816.00 | 8.000 | 816.00 | 8.000 | 816.00 | 8.000 | 816.00 | 8.000 |
| HFA 134a | 9307.19 | 91.25 | 9307.41 | 91.25 | 9307.52 | 91.25 | 9307.59 | 91.25 |
| Total (Nominal) | 10200.00 | 100.00 | 10200.00 | 100.00 | 10200.00 | 100.00 | 10200.00 | 100.00 |

The formulations were stored at 40 or 60ºC for two weeks. The amounts of the active ingredients and impurity levels were determined at 4, 7 and 14 days. All formulations showed favourable stability, although the best results were obtained with the low-water formulations, indicating that less water in the formulation is preferable.

## Claims

1. A solution formulation for nasal administration comprising azelastine, beclomethasone dipropionate, a co-solvent, hydrochloric acid and an HFA propellant, wherein the molar ratio of azelastine free base to hydrochloric acid is 6:1 to 15:1.

2. A formulation as claimed in claim 1, wherein the molar ratio of azelastine free base to hydrochloric acid is 9:1 to 12:1.

3. A formulation as claimed in any preceding claim, wherein the azelastine is present in the form of both azelastine free base and an azelastine salt.

4. A formulation as claimed in any preceding claim, wherein the co-solvent is ethanol.

5. A formulation as claimed in any preceding claim, wherein the HFA propellant is selected from 1,1,1,2-tetrafluoroethane (P134a), 1,1,1,2,3,3,3-heptafluoropropane (P227) and a mixture thereof.

6. A formulation as claimed in any preceding claim comprising azelastine, beclomethasone dipropionate, ethanol, hydrochloric acid and a propellant selected from 1,1,1,2-tetrafluoroethane (P134a), 1,1,1,2,3,3,3-heptafluoropropane (P227) and a mixture thereof, wherein the molar ratio of azelastine free base to hydrochloric acid is 9:1 to 10:9.

7. A formulation as claimed in any preceding claim for use in the prophylaxis and/or treatment of seasonal allergic rhinitis (including hay fever) and perennial rhinitis.

8. A nasal spray device comprising the pharmaceutical solution formulation as claimed in any preceding claim, for the delivery of the pharmaceutical solution to the nasal cavity in metered doses.

9. A nasal spray device as claimed in claim 8 comprising:
a pressurised aerosol canister including a vial containing the pharmaceutical solution, the aerosol canister further including a metering valve having a valve stem; and
an actuator for the aerosol canister, the actuator including a stem block having a receptacle into which the valve stem of metering valve of the aerosol canister is received and axially located and being displaceable relative to the vial of the aerosol canister to actuate the metering valve of the aerosol canister, a sump extending below the receptacle, the stem block further defining a discharge orifice for the pharmaceutical solution and a transfer channel through which a dispensed dose of the pharmaceutical solution is able to pass from the sump to the discharge orifice,
wherein the actuator further comprises a delivery outlet for the aerosol plume, the discharge orifice being arranged to direct the aerosol plume through the delivery outlet, and
wherein the axis of the delivery outlet may be perpendicular, or at an angle of up to 20º to the perpendicular, to the aerosol canister and the receptacle of the stem block.

## Patentansprüche

1. Eine Lösungsformulierung zur nasalen Verabreichung, bestehend aus Azelastin, Beclometason-Dipropionat, einem Hilfslösungsmittel, Salzsäure und einem HFA-Treibmittel, wobei das Molverhältnis von Azelastin Freie Base zu Salzsäure 6:1 bis 15:1 beträgt.

2. Eine Formulierung nach Anspruch 1, bei der das Molverhältnis von Azelastin freie Base zu Salzsäure 9:1 bis 12:1 beträgt.

3. Eine Formulierung nach einem der vorherigen Ansprüche, bei der Azelastin sowohl als freie Base als auch als Azelastinsalz vorliegt.

4. Eine Formulierung nach einem der vorherigen Ansprüche, bei der die Hilfslösung Ethanol ist.

5. Eine Formulierung nach einem der vorherigen Ansprüche, bei der das HFA-Treibmittel aus 1,1,1,2-Tetrafluorethan (P134a), 1,1,1,2,3,3,3-Heptafluorpropan (P227) und einer Mischung aus diesen gewählt wird.

6. Eine Formulierung nach einem der vorherigen Ansprüche, bestehend aus Azelastin, Beclometason-Dipropionat, Ethanol, Salzsäure und einem Treibmittel, das aus 1,1,1,2-Tetrafluorethan (P134a), 1,1,1,2,3,3,3-Heptafluorpropan (P227) und einer Mischung aus diesen gewählt wird, wobei das Molverhältnis von Azelastin freie Base zu Salzsäure 9:1 bis 10:9 beträgt.

7. Eine Formulierung nach einem der vorherigen Ansprüche zur Verwendung bei der Prophylaxe und/oder Behandlung saisonaler allergischer Rhinitis (einschließlich Heufieber) und ganzjähriger Rhinitis.

8. Eine Nasensprayvorrichtung, die die pharmazeutische Lösungsformulierung nach einem der vorherigen Ansprüche enthält, zur dosierten Abgabe der pharmazeutischen Lösung an die Nasenhöhle.

9. Eine Nasensprayvorrichtung nach Anspruch 8, umfassend:
einen unter Druck stehenden Aerosolbehälter mit einer Durchstechflasche, die die pharmazeutische Lösung enthält, wobei der Aerosolbehälter weiterhin ein Dosierventil mit einem Ventilschaft umfasst; und
einen Stellantrieb für den Aerosolbehälter, wobei der Stellantrieb einen Schaftblock mit einem Gefäß umfasst, in das der Ventilschaft des Dosierventils des Aerosolbehälters aufgenommen und axial gelagert wird und das in Bezug auf die Durchstechflasche des Aerosolbehälters verschiebbar ist, um das Dosierventil des Aerosolbehälters zu betätigen, einen Sammelschacht, der sich unter dem Gefäß erstreckt, wobei der Schaftblock weiterhin eine Auslassöffnung für die pharmazeutische Lösung und einen Transportkanal definiert, durch welchen eine zu verabreichende Dosis der pharmazeutischen Lösung vom Sammelschacht zur Auslassöffnung gelangen kann,
wobei der Stellantrieb weiterhin einen Abgabeauslass für den Aerosolnebel enthält, und die Auslassöffnung so eingerichtet ist, dass der Aerosolnebel durch den Abgabeauslass geleitet wird, und wobei die Achse des Abgabeauslasses lotrecht, oder in einem Winkel von bis zu 20⁰ zur Lotrechten, zum Aerosolbehälter und dem Gefäß des Schaftblocks sein kann.

## Revendications

1. Une formulation de solution pour administration nasale comprenant de l'azélastine, du dipropionate de béclométhasone, un co-solvant, de l'acide chlorhydrique et un agent propulseur HFA, dans laquelle le rapport molaire azélastine à base libre/acide chlorhydrique est de 6 :1 pour 15 :1.

2. Une formulation selon la revendication 1, dans laquelle le rapport molaire azélastine à base libre/acide chlorhydrique est de 9 :1 pour 12 :1.

3. Une formulation selon l'une quelconque des précédentes revendications, dans laquelle l'azélastine est présente à la fois sous forme d'azélastine à base libre et de sels issus de l'azélastine.

4. Une formulation selon l'une quelconque des précédentes revendications, dans laquelle le co-solvant est de l'alcool éthylique.

5. Une formulation selon l'une quelconque des précédentes revendications, dans laquelle l'agent propulseur HFA est sélectionné à partir d'un groupe composé du 1,1,1,2-tétrafluoroéthane (P134a), du 1,1,1,2,3,3,3-heptafluoropropane (P227) et d'un mélange de ceux-ci.

6. Une formulation selon l'une quelconque des précédentes revendications comprenant de l'azélastine, du dipropionate de béclométhasone, de l'alcool éthylique, de l'acide chlorhydrique et un agent propulseur sélectionné à partir d'un groupe composé du 1,1,1,2-tétrafluoroéthane (P134a), du 1,1,1,2,3,3,3-heptafluoropropane (P227) et d'un mélange de ceux-ci, dans laquelle le rapport molaire azélastine à base libre/acide chlorhydrique est de 9 :1 pour 10 :9.

7. Une formulation selon l'une quelconque des précédentes revendications destinée à être utilisée dans la prophylaxie et/ou le traitement de la rhinite allergique saisonnière (y compris le rhume des foins) et de la rhinite pérenniale.

8. Un vaporisateur nasal comprenant la formulation de solution pharmaceutique selon l'une quelconque des précédentes revendications, pour une administration de la solution pharmaceutique dans les fosses nasales de façon dosée.

9. Un vaporisateur nasal selon la revendication 8 comprenant :
un récipient à aérosol pressurisé incluant une ampoule contenant la solution pharmaceutique, le récipient à aérosol incluant également une valve doseuse possédant une tige ; et un actionneur pour le récipient à aérosol, l'actionneur incluant une tige de blocage possédant un réceptacle dans lequel la tige de la valve doseuse du récipient à aérosol est réceptionnée, axialement fixée et déplaçable par rapport à l'ampoule du récipient à aérosol pour actionner la valve doseuse du récipient à aérosol, un puisard s'étendant au-dessous du réceptacle, la tige de blocage délimitant également un orifice d'évacuation pour la solution pharmaceutique et une voie de transfert à travers laquelle peut passer une dose de la solution pharmaceutique délivrée depuis le puisard vers l'orifice d'évacuation,
dans lequel l'actionneur comprend également une sortie d'évacuation pour les panaches d'aérosols, l'orifice d'évacuation étant placé de façon à diriger les panaches d'aérosols vers la sortie d'évacuation, et dans lequel l'axe de la sortie d'évacuation peut être perpendiculaire (ou à un angle de 20° maximum par rapport à la perpendiculaire) au récipient à aérosol et au réceptacle de la tige de blocage.
